# EUROPEAN PATENT APPLICATION

(11) **EP 3 210 975 A1**
(43) Date of publication of application: **30.08.2017**
(21) Application number: 16382075.6
(22) Date of filing: 24.02.2016
(51) Int. Cl.: C07D 223/16, A61K 31/5513, A61P 3/04

(54) **COCRYSTALS OF LORCASERIN**

(71) Applicant: Enantia, S.L., 08028 Barcelona (ES)
(72) Inventor: TESSON, Nicolas, 08902 L'HOSPITALET DE LLOBREGAT (ES); GORDO CAMPÓN, Esther, 08020 BARCELONA (ES)
(74) Representative: ZBM Patents - Zea, Barlocci & Markvardsen

(57) **Abstract**

Cocrystals of Lorcaserin hydrochloride and an organic diacid, in particular with (+)-di-p-toluoyl-D-tartaric acid; (+)-camphoric acid; and oxalic acid, processes for their preparation, their therapeutical indications, as well as, pharmaceutical compositions containing them. It also relates to a resolution process of lorcaserin which goes through the formation of the cocrystal of (R)-Lorcaserin hydrochloride with (+)-di-p-toluoyl-D-tartaric.

## Description

The present invention relates to cocrystals of Lorcaserin, processes for their preparation, and their use as medicaments. It also relates to pharmaceutical compositions comprising them. The invention also relates to the use of the cocrystals as intermediates for the preparation of Lorcaserin.

### BACKGROUND ART

Lorcaserin is the International Nonproprietary Name (INN) of (1*R*)-8-chloro-1-methyl-2,3,4,5-tetrahydro-1*H*-3-benzazepine. It is a selective 5-HT_{2C} receptor agonist. The activation of 5-HT_{2C} receptors in the hypothalamus is supposed to activate proopiomelanocortin (POMC) production and consequently promote weight loss through satiety. Lorcaserin, currently marketed under the trade name Belviq, is a weight-loss drug developed by Arena Pharmaceuticals. It has serotonergic properties and acts as an anorectic.

The structure of Lorcaserin corresponds to formula (I) below.

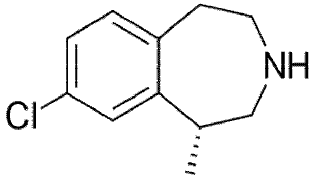

Lorcaserin is a chiral compound which was first disclosed in the patent family of EP1411881B1. This document discloses a synthesis of Lorcaserin in racemic form. It also discloses an enantiomeric resolution process via chiral chromatography of the trifluoroacetamide protected amine Lorcaserin and the subsequent hydrolysis of the separated enantiomers to afford Lorcaserin (see Example 55).

Several resolution processes for the preparation of Lorcaserin and/or its hydrochloride salt have been disclosed in the art. Thus, the patent EP2189448B1 discloses a process for the resolution of Lorcaserin free base with 0.25 eq. of L-tartaric acid in t-butanol/water (see Examples 4 and 13) and a process for the preparation of the hydrochloride salt of Lorcaserin (see Example 14). Example 4 afforded Lorcaserin hemitartrate salt. The use of this t-butanol/water as solvent system resulted in the crystallization of the crude hemitartrate with approximately 68-80% ee. An additional recrystallization was required to increase the % ee and as a result a decreased in the yield was obtained. The singly recrystallized hemitartrate was obtained with 98.9% ee and the reported yield after two steps was 48%. However the calculated yield gives only 30%. Example 13 afforded the tartrate salt of Lorcaserin with 98.7% ee (yield: 24%). The reproduction of this resolution process (example 13) by LEK Pharmaceuticals in the patent application WO2015007897A1 seems to afford poor results (98.7% ee but with a yield of only 15%).

US8168782B2 discloses an improved process for the resolution of the racemic Lorcaserin with L-tartaric acid. The resolution is performed using 0.25 eq. of L-tartaric acid in acetone/water (see example 3). In this example the hemitartrate of Lorcaserin was obtained with an overall yield of 27.1% and 99.7% ee (two steps). It is described that the use of acetone in the salt forming step, instead of t-BuOH as described in EP2189448B1, eliminates the need for an additional recrystallization and has a direct result on improving the yield. The present applicant has tried to reproduce the example 3 of this patent at a lower scale. Although following accurately all the process and using the specific molar ratio of racemic Lorcaserin, acetone, L-tartaric acid and water described in the patent, a racemic salt of stoichiometry 2:1 was obtained instead of the described diastereomeric salt.

Finally, CN103755635 discloses a resolution process based on the crystallization of diastereomeric salts of Lorcaserin free base and some derivatives of tartaric acid such as D-(+)-dibenzoyl tartaric acid and D-(+)-diacetyl tartaric acid.

On the other hand, EP2327698B1 discloses several crystalline forms of Lorcaserin hydrochloride, named Form I, Form II and Form III. Form I and Form II are anhydrous forms, however they are hygroscopic forms and readily convert to Form III upon exposition to moisture. WO2011153206A1 discloses another crystalline form of Lorcaserin hydrochloride named Form IV. It is said that is the most stable of the known anhydrous polymorphs of Lorcaserin hydrochloride, although it is not thermodynamically favoured over the hydrate Form III at % relative humidity (RH) ≥ 20. The conversion of the lorcaserin hydrochloride Form IV to the hemihydrate Form III upon exposure to moisture is also disclosed.

According to WO2014135545A1 lorcasern hydrochloride Form III has the drawback that it can convert (at least partially) to another form, such as Form II, under particular environmental conditions, for example when exposed to elevated temperatures as explained in WO2011153206A1. Example 5 of WO2011153206A1 discloses the thermodynamic relationships between Lorcaserin hydrochloride salt hemihydrate Form III and the anhydrous polymorphs of Lorcaserin hydrochloride salt. It is said that at a critical water activity (aw) a given anhydrous form and Lorcaserin hydrochloride salt hemihydrate will have equal solubility and can coexist in equilibrium. Below the critical aw, the anhydrous form is described to be more thermodynamically stable, thus less soluble. Above the critical water activity Lorcaserin hydrochloride salt hemihydrate Form III is described to be more thermodynamically stable, thus less soluble. The water activity dependent equilibrium of Lorcaserin hydrochloride hemihydrate Form III and the anhydrous polymorphs of Lorcaserin hydrochloride salt can be a drawback for manufacturing a pharmaceutical composition based on Lorcaserin hydrochloride Form III, as the pharmaceutical compositions comprising the desired Form III might not be obtained reliably or might not be stable upon storage with regard to the polymorphic form.Thus, the known crystalline forms of Lorcaserin hydrochloride including Form III can be prone to polymorphic conversion when exposed to an environment having a higher relative humidity and/or an elevated temperature such as for example the one needed for melt granulation.

Different solid forms of a pharmaceutically active ingredient can have different characteristics, and offer certain advantages, for example with regard to solubility or bioavailability. Thus, the discovery of new solid forms allows for improving the characteristics of the pharmaceutical formulations of the active ingredients, since some forms are more adequate for one type of formulation, and other forms for other different formulations. Furthermore, depending on the therapeutic indications, one or another pharmaceutical formulation may be preferred.

Therefore, there is still the need to find efficient routes for the preparation of Lorcaserin hydrochloride, as well as, an ongoing need for new or crystalline forms of Lorcaserin hydrochloride.

### SUMMARY OF THE INVENTION

Inventors have found that Lorcaserin hydrochloride can form cocrystals with organic diacids such as (+)-camphoric acid, oxalic acid, or (+) di-p-toluoyl-D-tartaric acid. The formation of different cocrystals can allow modulating stability, solubility, and hygroscopicity, and can provide enhanced bioavailability, enhanced galenic properties, or higher purity. Cocrystals have been demonstrated to be complementary or, at least, equal to salts for purification purposes, so products can be isolated using cocrystallization processes in order to obtain the desired specifications. While active pharmaceutical ingredients (APIs) have been recognized to form crystalline polymorphs, solvates, hydrates and amorphous forms, for a number of years, there is little knowledge about which ones can form cocrystals. By cocrystallizing an API or a salt of an API with a coformer (the second component of the cocrystal), a new solid state form of the API is created having unique properties compared with existing solid forms of the API or its salts. However, cocrystal formation is not predictable, and in fact is not always possible. Moreover, there is no way to predict the properties of a particular cocrystal of a compound until it is formed. Finding the right conditions to obtain a particular cocrystal can take significant time, effort, and resources.

In the particular case of Lorcaserin, there is neither a suggestion in the prior art regarding the possibility that Lorcaserin hydrochloride could form cocrystals with a second component, even less, that the second component may be an organic diacid, nor an indication about how to prepare any possible cocrystal of Lorcaserin.

Accordingly, an aspect of the present invention relates to the provision of a cocrystal of Lorcaserin hydrochloride and a organic diacid.

Another aspect of the present invention relates to a cocrystal of Lorcaserin hydrochloride and an organic diacid for use as a medicament.

Another aspect of the present invention relates to a cocrystal of Lorcaserin hydrochloride and an organic diacid for use in the prevention and/or treatment of obesity.

Another aspect of the present invention relates to a pharmaceutical composition comprising a cocrystal of Lorcaserin hydrochloride and an organic diacid, together with appropriate amounts of pharmaceutical excipients or carriers.

Another aspect of the present invention relates to a resolution process of Lorcaserin which goes through the formation of a cocrystal of (R)-Lorcaserin hydrochloride with (+)-di-p-toluoyl-D-tartaric acid.

Finally, it is also part of the invention the processes for preparing specific cocrystals of (R)-Lorcaserin hydrochloride with organic diacids, in particular, (+)-camphoric acid, oxalic acid, and (+) di-p-toluoyl-D-tartaric acid. The processes comprising contacting either (R)-Lorcaserin hydrochloride, or racemic Lorcaserin free base with the corresponding organic diacid in a suitable solvent system under suitable conditions, and isolating the corresponding cocrystals.

### BRIEF DESCRIPTION OF THE DRAWINGS

FIG. 1 shows the XRPD of the cocrystal of Lorcaserin hydrochloride with (+)-di-p-toluoyl-D-tartaric acid named Form A

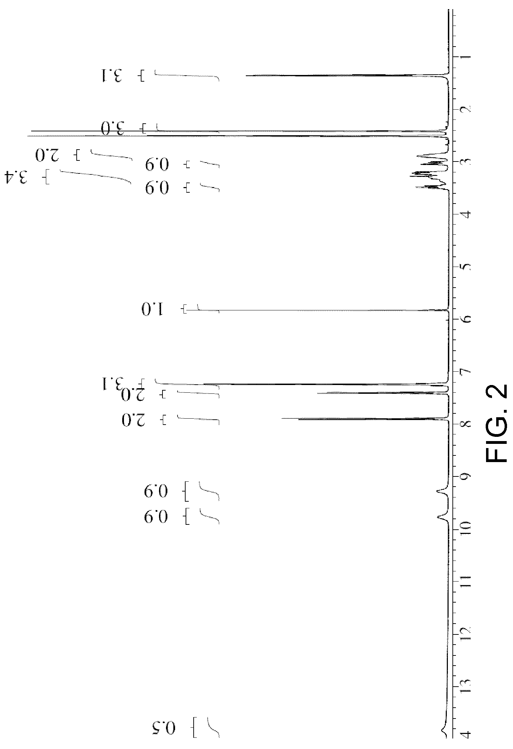

FIG. 2 shows 1 H NMR of the Form A

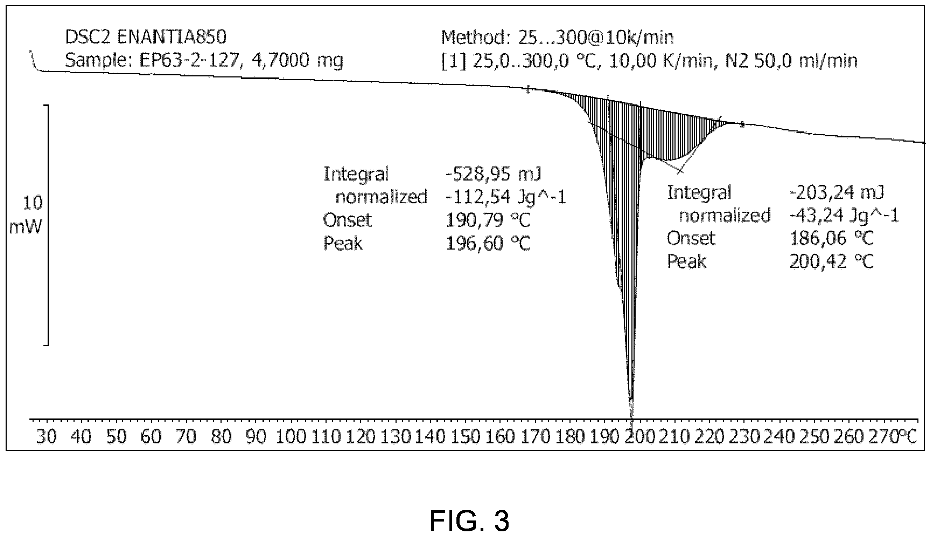

FIG.3 shows the DSC of the Form A
FIG. 4 shows the XRPD of the cocrystal of (R)-Lorcaserin hydrochloride with (+)-camphoric acid named Form B

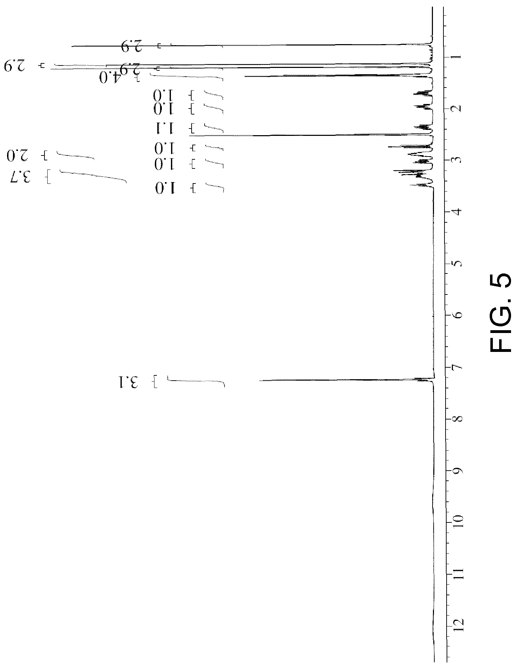

FIG. 5 shows 1 H NMR of the Form B

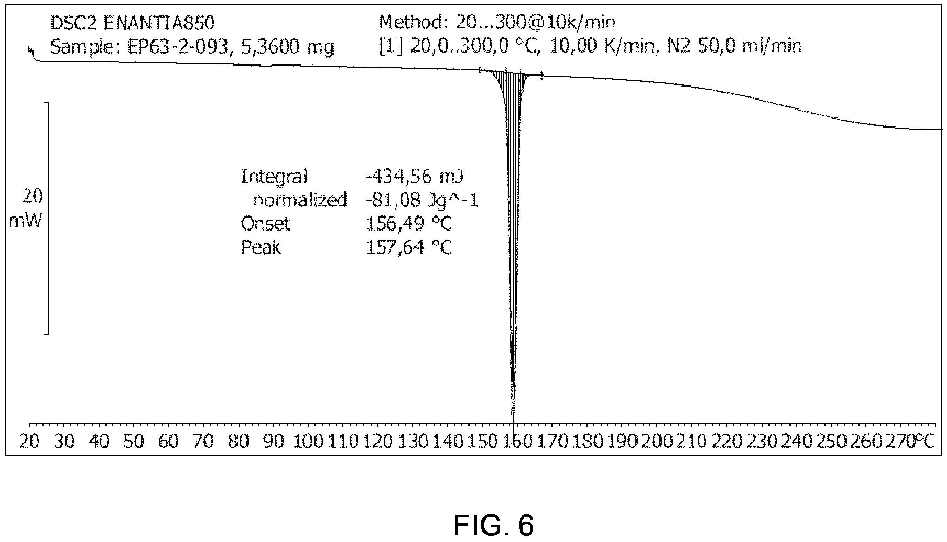

FIG. 6 shows the DSC of the Form B
FIG. 7 shows the XRPD of the cocrystal of (R)-Lorcaserin hydrochloride with oxalic acid named Form C

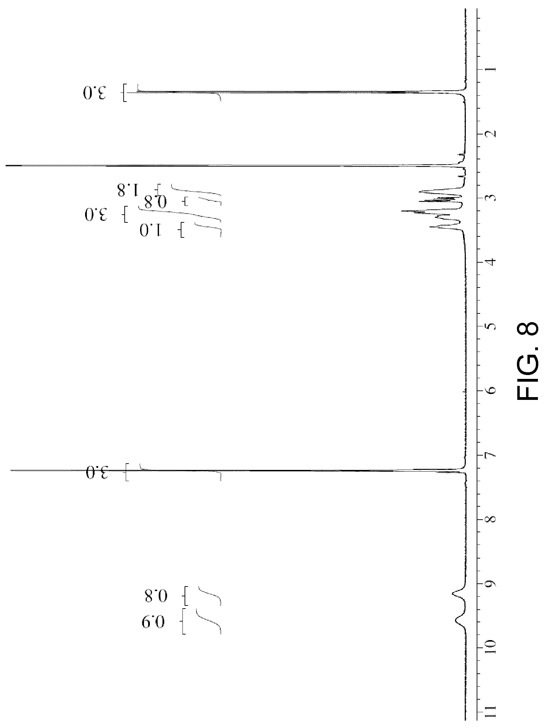

FIG. 8 shows 1 H NMR of the Form C

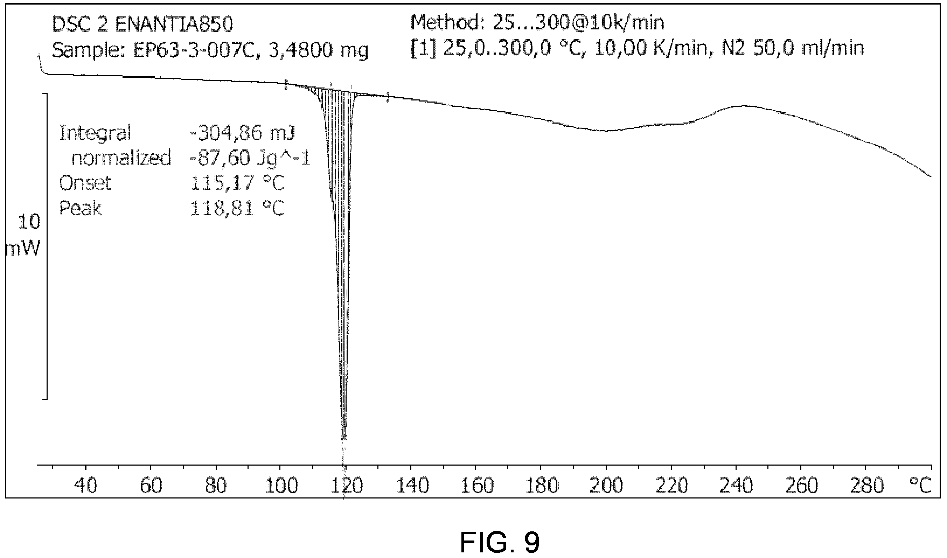

FIG.9 shows the DSC of the Form C

### DETAILED DESCRIPTION OF THE INVENTION

For the sake of understanding, the following definitions are included and expected to be applied throughout description, claims and drawings.

Unless stated otherwise, the term "lorcaserin" in the context of the present invention relates to (R)-lorcaserin. Both terms have been used interchangeably.

The term "cocrystal" refers herein to a crystalline entity with at least two different components constituting the unit cell at room temperature (20-25 °C) and interacting by weak interactions. Thus, in a cocrystal the active pharmaceutical ingredient crystallizes with one or more neutral components. The cocrystals may include one or more solvent molecules in the crystal lattice.

The term "weak interaction" refers herein as an interaction which is neither ionic nor covalent, and includes for example: hydrogen bonds, van der Waals interactions, and π-π stacking.

The term "solvate" is to be understood as meaning any form of the cocrystal in which the compound has attached to it via non-covalent binding solvent molecules. When the solvent is water the solvate is a hydrate.

When a ratio of components of the cocrystals of the invention is specified it refers to the molar ratio between Lorcaserin hydrochloride and a further component that forms the cocrystal. The term "molar ratio" has been used to express the stoichiometric amount in mols of each of the components of a cocrystal.

When values of characteristic peaks of an X-ray diffractogram are given it is said that are "approximate" values. It should be understood that the values are the ones shown in the corresponding lists or tables ± 0.3 degrees 2 theta measured in an X-ray diffractometer with Cu-K_{α} radiation λ=1.5406 Å.

The term "room temperature" as disclosed herein refers to a temperature of the environment, without heating or cooling, and is generally comprised of from 20 to 25 °C.

For the purposes of the invention, any ranges given include both the lower and the upper end-points of the range. Ranges given, such as temperatures, times, and the like, should be considered approximate, unless specifically stated.

For the term "pure crystalline form" is understood that the crystallline form is substantially free of any other crystalline forms. The expression "substantially free of other crystalline forms" is to be understood to mean that the cocrystals of the invention show an XRPD with the characteristic peaks mentioned below for each cocrystal but without having any other significant peak of a subject compound which is an organic or inorganic compound in crystalline form. More precisely, the term pure crystalline form means that the crystalline form has a percentage by weight equal to or lower than 7% of any other crystalline form, preferably, equal to or lower than 5% by weight, more preferably, equal to or lower than 1 % by weight.

Enantiomeric excess (ee) is a measurement of purity used for chiral substances. It reflects the degree to which a sample contains one enantiomer in greater amounts than the other.The term "enantiomeric purity" (or optical purity) is defined as the fractional excess of one enantiomer over the other.

The expression "cocrystal obtainable by" is used here to define each specific cocrystal of the invention by the process for obtaining it and refers to the product obtainable by any of the corresponding processes disclosed herein. For the purposes of the invention the expressions "obtainable", "obtained" and equivalent expressions are used interchangeably, and in any case, the expression "obtainable" encompasses the expression "obtained".

The terms "wet grinding" and "liquid assisted grinding" are equivalent and refer to a technique which consists of milling or grinding the product or mixture with some drops of solvent added. Neat and liquid-assisted grinding are techniques that can be employed in order to produce cocrystals. In neat (dry) grinding, cocrystal formers are ground together manually using a mortar and pestle, using a ball mill, or using a oscillatory mill. In liquid-assisted grinding, or kneading, a small amount of liquid (solvent), for instance, some drops of liquid, is added to the grinding mixture.

The term "therapeutically effective amount" as used herein, refers to the amount of a compound that, when administered, is sufficient to prevent development of, or alleviate to some extent, one or more of the symptoms of the illness to be treated. The particular dose of compound administered according to this invention will of course be determined by the particular circumstances surrounding the case, including the compound administered, the route of administration, the particular condition being treated, and the similar considerations.

The term "pharmaceutical composition" refers to a mixture of a compound disclosed herein with other chemical components, such as diluents or carriers. The pharmaceutical composition facilitates administration of the compound to an organism.

The term "pharmaceutically acceptable excipients or carriers" refers to pharmaceutically acceptable material, composition or vehicle, such as liquid or solid filler, diluent, excipient, solvent, or encapsulating material. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The term "treatment" meant to include alleviating or eradicating a disorder, disease, or condition, or one or more of the symptoms associated with the disorder, disease or condition, or alleviating or eradicating the cause(s) of the disorder, disease, or condition itself.

As mentioned above, it is provided a cocrystal of Lorcaserin hydrochloride and an organic diacid.The organic diacid may be a pharmaceutically acceptable organic diacid, which in the context of the present invention are those acids which are, within the scope of sound medical judgement, suitable for use in contact with the tissues of human and lower animals without undue toxicity, irritation, allergic response and the like, commensurate with a reasonable benefit/risk ratio, and effective for their intended use. In a particular embodiment, the organic diacids are those where the acid groups are separated by among 0-6 carbon atoms. Preferably, the organic diacids are those where the acid groups are separated by among 0-3 carbon atoms.

In a preferred embodiment, any of the cocrystals of the invention are substantially free of any other crystalline forms. They may exist in an anhydrous and/or solvent-free form or as a hydrate and/or solvate.

Preferred cocrystals are those which can be readily prepared, easy to scale-up, have acceptable shelf-life and are in form accepted for use in pharmaceutical formulations. The cocrystals of the invention may also be useful as intermediates in the preparation of lorcaserin or its pharmaceutically acceptable salts such as the hydrochloride salt, in high purity.

Preferred cocrystals according to the invention are the following:
- Cocrystal of (R)-Lorcaserin hydrochloride with (+)-di-p-toluoyl-D-tartaric acid (D-DTTA);
- Cocrystal of (R)-Lorcaserin hydrochloride with (+)-camphoric acid; and
- Cocrystal of (R)-Lorcaserin hydrochloride with oxalic acid.

The specific crystalline forms of the cocrystals of the present invention have been characterized by X-ray powder diffraction (XRPD), proton nuclear magnetic resonance analyses (¹H NMR), and differential scanning calorimetry (DSC).

Diffraction measurements described herein were performed at ambient conditions on a PANalytical X'Pert PRO diffractometer with reflection θ-θ geometry, equipped with Cu K-alpha radiation and a PIXcel detector, operated at 45 kV and 40 mA. Each powder was mounted on a zero-background silicon holder and allowed to spin during the data collection at 0.25 rev/s. The measurement angular range was 3.0-40.0° (2θ) with a step size of 0.013°. The scanning speed was 0.32826°/s.

¹H NMR analysis were recorded in deuterated dimethyl sulfoxide in a Varian Mercury 400 spectrometer, equipped with a broadband probe ATB 1 H/19F/X of 5 mm. Spectra were acquired dissolving 5-10 mg of sample in 0.6 mL of deuterated solvent.

DSC analyses were performed with a Mettler Toledo DSC2. Each sample was weighed into a 40 µL aluminium crucible with a pinhole lid and heated from 30 to 300 °C at a rate of 10 °C/min, under nitrogen (50 mL/min).

The cocrystals of Lorcaserin hydrochloride and a organic diacid can have different molar ratios between the Lorcaserin hydrochloride and the further component. Preferably, the molar ratio of (R)-Lorcaserin hydrochloride / organic diacid is 1:1 or 2:1.

The ratio (R)-Lorcaserin hydrochloride organic diacid in the cocrystal may be determined by ¹H NMR, titration, or elemental analysis.

In a particular embodiment the cocrystal of Lorcaserin hydrochloride and a organic diacid is a cocrystal of (R)-Lorcaserin hydrochloride with (+)-di-p-toluoyl-D-tartaric acid.

In a preferred embodiment of the invention, the cocrystal of (R)-Lorcaserin hydrohchloride with (+)-di-p-toluoyl-D-tartaric acid has a molar ratio of (R)-Lorcaserin hydrochloride /(+)-di-p-toluoyl-D-tartaric acid of 2:1.

In a more preferred embodiment the cocrystal of (R)-Lorcaserin hydrochloride with (+)-dip-toluoyl-D-tartaric acid is characterized by having an X-ray diffractogram that comprises characteristic peaks at approximately 6.8 and 13.5 degrees 2 theta (Cu-K_{α} radiation, λ = 1.5406 Å). This specific cocrystal form is named herein cocrystal Form A. In a still more preferred embodiment, the cocrystal Form A is characterized by having an X-ray diffractogram that further comprises characteristic peaks at approximately 10.7 and 17.6 degrees 2 theta (Cu-K_{α} radiation, λ = 1.5406 Å). More specifically, this new cocrystal Form A is characterized by exhibiting in the powder X-ray diffractogram a pattern of peaks, expressed in 2 theta units in degrees, 2θ (°), which is shown in Table 1.

**Table 1: List of selected peaks of XRPD (only peaks with relative intensity greater than or equal to 1% are indicated):**

| Pos. | Rel. Int. |
|---|---|
| [°2Th.] | [%] |
| 6,8 | 62 |
| 10,0 | 13 |
| 10,7 | 7 |
| 12,4 | 27 |
| 13,5 | 100 |
| 14,0 | 33 |
| 14,3 | 10 |
| 15,7 | 7 |
| 16,1 | 7 |
| 16,7 | 7 |
| 17,6 | 99 |
| 18,2 | 4 |
| 18,6 | 78 |
| 21,0 | 25 |
| 21,4 | 23 |
| 21,6 | 20 |
| 22,1 | 9 |
| 22,4 | 7 |
| 23,1 | 9 |
| 23,7 | 70 |
| 24,2 | 66 |
| 24,6 | 12 |
| 25,0 | 10 |
| 25,2 | 7 |
| 25,8 | 8 |
| 26,2 | 6 |
| 26,7 | 2 |
| 27,3 | 32 |
| 27,5 | 23 |
| 27,9 | 19 |
| 28,3 | 6 |
| 29,0 | 14 |
| 29,8 | 13 |
| 30,3 | 5 |
| 30,6 | 10 |
| 31,4 | 18 |
| 31,9 | 9 |
| 32,1 | 10 |
| 32,7 | 3 |
| 34,7 | 8 |
| 35,2 | 4 |
| 35,7 | 5 |
| 36,4 | 3 |
| 36,9 | 4 |
| 37,7 | 5 |
| 38,0 | 4 |
| 39,0 | 4 |

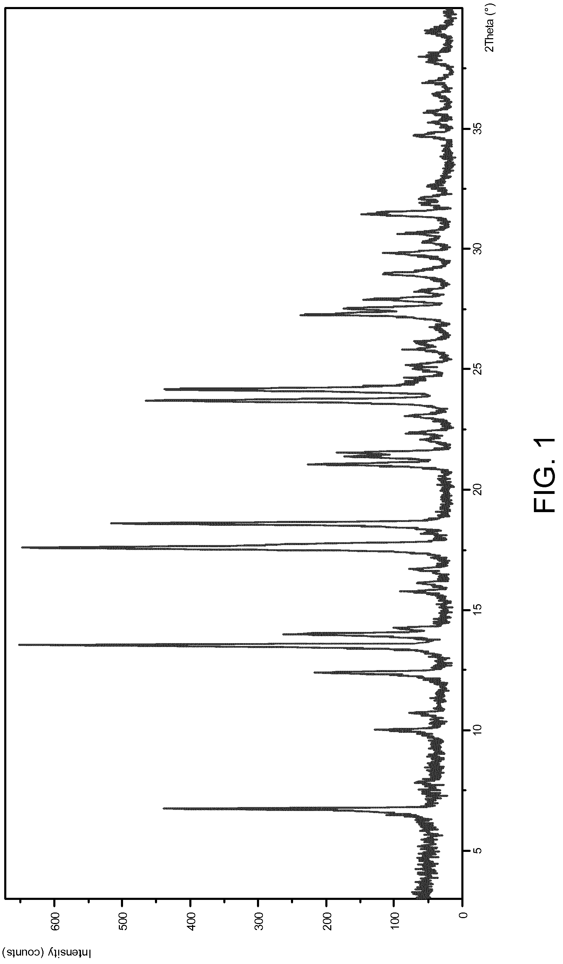

This cocrystal Form A may be further characterized by an X-ray diffractogram as in FIG. 1.

This cocrystal Form A may also be further characterized by the following ¹H NMR spectra (DMSO-d6, 400 MHz): δ = 13.83 (s broad, 0.5H), 9.76 (s broad, 1 H), 9.28 (s broad, 1 H), 7.90 (d, J= 8.2 Hz, 2H), 7.40 (d, J= 8.2 Hz, 2H), 7.28-7.20 (m, 3H), 5.83 (s, 1 H), 3.54-3.42 (m, 1 H), 3.42-3.15 (m, 3H), 3.02(dd, J= 15.6 Hz, 7.0 Hz, 1 H), 2.95-2.81 (m, 2H), 2.400 (s, 3H) and 1.35 (d, J= 7.0 Hz, 3H).

This cocrystal Form A may also be further characterized by a DSC curve which shows two endothermic peaks. The first one, with an onset at approximately 190.8°C is probably caused by the melting of the product. The form of that peak, with a shoulder at the beginning, can be due to the overlapping of another endothermic event. The second one, with an onset at 186.1 °C is probably caused by the degradation of the compound.

This cocrystal of (R)-Lorcaserin hydrochloride with (+)-di-p-toluoyl-D-tartaric acid may be prepared by a preparation process which comprises crystallizing/precipitating the cocrystal from a mixture of racemic Lorcaserin hydrochloride or (R)-Lorcaserin·HCl, (+)-dip-toluoyl-D-tartaric acid, and a solvent system selected from the group consisting of: methylethylketone/water, acetone/water, isobutanol, n-butanol, ethyl acetate, and methylisobutylketone saturated of water; and isolating the solid thus obtained.

It can also be prepared by a process comprising slurrying racemic Lorcaserin hydrochloride or (R)-Lorcaserin hydrochloride with (+)-di-p-toluoyl-D-tartaric acid in ethyl acetate at room temperature; and isolating the solid thus obtained.

Depending on the method and solvent system, one or two equivalent of (+)-di-p-toluoyl-D-tartaric acid may be necessary. However, other amounts can also be used. For instance, in a particular embodiment, in isobutanol the stoichiometry of racemic Lorcaserin hydrochloride and (+)-di-p-toluoyl-D-tartaric acid is 4:3.

These processes afford the cocrystal of (R)-Lorcaserin hydrochloride with (+)-di-p-toluoyl-D-tartaric acid Form A with 31-46% yield and an enantiomeric excess between 70 and 90.

When necessary, one or two recrystallizations of the cocrystal of (R)-Lorcaserin hydrochloride with (+)-di-p-toluoyl-D-tartaric acid Form A are carried out in a solvent system selected from isobutanol, isobutanol/antisolvent, n-butanol,and n-butanol/antisolvent. The antisolvent is selected from the group consisting of isobutyl acetate, heptane, methylisobutylketone, tert-butylmethylether, and mixtures thereof.

Thus, after recrystallizing twice the cocrystal of (R)-Lorcaserin hydrochloride with (+)-di-p-toluoyl-D-tartaric acid Form A in the solvents mentioned above, the cocrystal Form A is obtained with good yield and high %ee. The overall yield is 30% and the enantiomeric excess ≥ 99%. In a preferred embodiment, the recrystallization is carried out with a mixture of isobutanol/heptane.

The cocrystal of (R)-Lorcaserin hydrochloride with (+)-di-p-toluoyl-D-tartaric acid Form A of the invention may also be defined by its preparation process. Accordingly, this aspect of the invention can be formulated as cocrystal of (R)-Lorcaserin hydrochloride with (+)-di-p-toluoyl-D-tartaric acid Form A as defined above, obtainable by any of the previous processes, optionally, including any preferred or particular embodiment of the processes and possible combinations of some of the process features disclosed above.

Since this cocrystal form is obtained with an enantiomeric excess ≥ 99%, it is useful as an intermediate for the resolution of of racemic Lorcaserin hydrochloride. Thus, the method of resolution of racemic Lorcaserin hydrochloride by formation of a cocrystal of (R)-Lorcaserin hydrochloride with (+)-di-p-toluoyl-D-tartaric acid is also part of the invention.

The process of resolution from racemic Lorcaserin hydrochloride comprises: a) preparing a cocrystal of (R)-Lorcaserin hydrochloride with (+)-di-p-toluoyl-D-tartaric acid Form A by a process which comprises either crystallizing/precipitating the cocrystal from a mixture of racemic Lorcaserin hydrochloride, (+)-di-p-toluoyl-D-tartaric acid, and a solvent system selected from the group consisting of: methylethylketone/water, acetone/water, isobutanol, n-butanol, ethyl acetate, and methylisobutylketone saturated of water; followed by isolating the solid thus obtained; and then followed by carrying out one or two recrystallizations in a solvent system selected from the group consisting of isobutanol, isobutanol/antisolvent, n-butanol, and n-butanol/antisolvent wherein the antisolvent is selected from the group consisting of isobutyl acetate, heptane, methylisobutylketone, tert-butyl methyl ether and mixtures thereof; b) slurrying the cocrystal in a biphasic solvent system selected from the group consisting of ethyl acetate/water, and methylisobutylketone/water to dissociate the cocrystal Form A; and c) isolating (R)-Lorcaserin hydrochloride from the aqueous phase. The process also allows recovering the (+)-di-p-toluoyl-D-tartaric acid from the organic phase.

The isolation step may comprise the crystallization of (R)-Lorcaserin hydrochloride from the aqueous solution containing it, optionally, in the presence of an antisolvent.

The isolation step may also be carried out by basifying the aqueous solution of (R)-Lorcaserin hydrochloride to obtain a solution of (R)-Lorcaserin free base and then extracting the (R)-Lorcaserin free base with one or more extractions in ethyl acetate or methyl isobutyl ketone. The organic solution containing (R)-Lorcaserin free base can be used to crystallize (R)-Lorcaserin hydrochloride in presence of hydrochloric acid or to crystallize a cocrystal of (R)-Lorcaserin hydrochloride in presence of hydrochloric acid and a coformer such as (1 R, 3S)-(+)-camphoric acid or oxalic acid yielding (R)-Lorcaserin hydrochloride, Form B or Form C respectively. The compounds thus obtained may be isolated, for instance, by filtration.

Advantageously, the results of the resolution process are better than the resolution process described in the patent US8168782B2 from racemic Lorcaserin free base. In addition, the use of racemic Lorcaserin hydrochloride instead of Lorcaserine free base allows to introduce a purification step before the resolution steps affording a more robust process. Furthermore, it is easy to recover the resolution agent to be used for another resolution process. Consequently, this improved process has better industrial applicability and utility than the known processes and overcome the drawbacks of the existing ones.

The (R)-Lorcaserin hydrochloride before or after being isolated from the aqueous solution of step b) of the resolution process disclosed above can be used to prepare different crystalline forms with high enantiomeric purity. Thus, it is also part of the invention the use of the cocrystal of (R)-Lorcaserin hydrochloride with (+)-di-p-toluoyl-D-tartaric acid Form A as an intermediate for the preparation of (R)-Lorcaserin or a salt thereof such as the hydrochloride salt, or of cocrystals of (R)-Lorcaserin hydrochloride and a organic diacid, with high enantiomeric purity, in particular, with 99%ee.

In addition, any of the crystalline forms of the present invention may be obtained in pure crystalline form, i.e. in a form which is substantially free of other crystalline forms by the processes described herein.

In a particular embodiment the cocrystal of Lorcaserin hydrochloride and a organic diacid is a cocrystal of (R)-Lorcaserin•HCl with (+)-camphoric acid.

In a preferred embodiment of the invention, the cocrystal of (R)-Lorcaserin hydrochloride with (+)-camphoric acid has a molar ratio of (R)-Lorcaserin hydrochloride/(+)-camphoric acid of 1:1.

In a more preferred embodiment the cocrystal of (R)-Lorcaserin hydrochloride with (+)-camphoric acid is characterized by having an X-ray diffractogram that comprises characteristic peaks at approximately 14.2 and 18.7 degrees 2 theta (Cu-K_{α} radiation, λ = 1.5406 Å). This specific cocrystal form is named herein cocrystal Form B. In a still more preferred embodiment, the cocrystal B is characterized by having an X-ray diffractogram that further comprises characteristic peaks at approximately 15.4 and 18.0degrees 2 theta (Cu-K_{α} radiation, λ = 1.5406 Å). More specifically, this new cocrystal Form B is characterized by exhibiting in the powder X-ray diffractogram a pattern of peaks, expressed in 2 theta units in degrees, 2θ (°), which is shown in Table 2.

**Table 2: List of selected peaks of XRPD (only peaks with relative intensity greater than or equal to 1% are indicated):**

| Pos. | Rel. Int. |
|---|---|
| [°2Th.] | [%] |
| 10,7 | 9 |
| 12,8 | 11 |
| 13,1 | 48 |
| 14,2 | 48 |
| 14,9 | 18 |
| 15,4 | 100 |
| 16,1 | 9 |
| 16,5 | 14 |
| 17,0 | 33 |
| 17,8 | 21 |
| 18,0 | 18 |
| 18,7 | 72 |
| 19,5 | 16 |
| 19,8 | 10 |
| 20,8 | 11 |
| 21,6 | 4 |
| 21,9 | 4 |
| 22,3 | 4 |
| 22,6 | 6 |
| 23,1 | 8 |
| 24,1 | 8 |
| 24,9 | 3 |
| 25,1 | 7 |
| 25,3 | 6 |
| 25,8 | 9 |
| 26,0 | 7 |
| 26,4 | 11 |
| 27,0 | 8 |
| 27,3 | 8 |
| 27,7 | 4 |
| 28,4 | 4 |
| 28,5 | 3 |
| 29,8 | 4 |
| 30,1 | 8 |
| 30,4 | 8 |
| 31,7 | 7 |
| 31,8 | 3 |
| 32,6 | 9 |
| 33,7 | 2 |
| 34,4 | 4 |
| 34,9 | 4 |
| 36,2 | 3 |
| 36,8 | 4 |

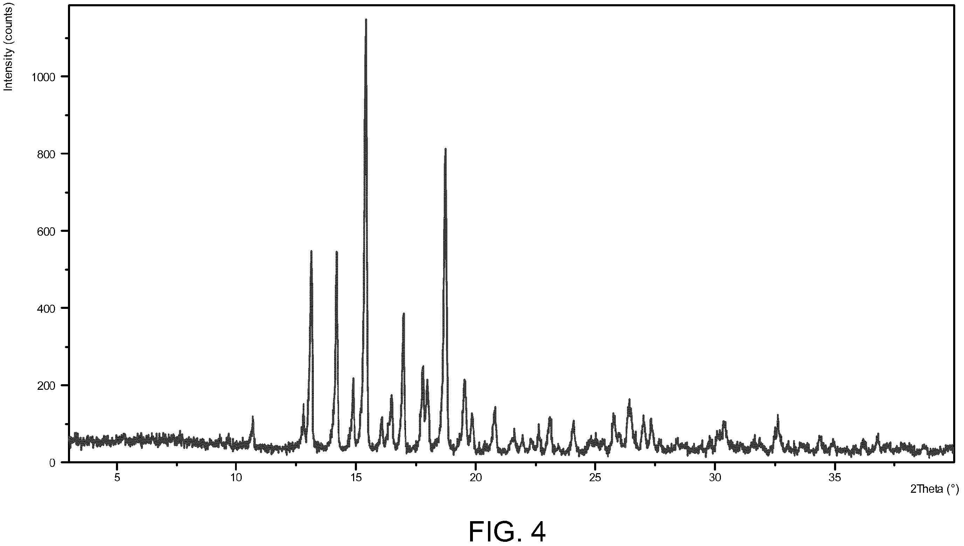

The cocrystal Form B may be further characterized by an X-ray diffractogram as in FIG. 4.

The cocrystal Form B may also be further characterized by the following ¹H NMR (DMSO-d6, 400 MHz): δ = 7.29-7.20 (m, 3H), 3.55-3.42 (m, 1 H), 3.42-3.16 (m, 3H), 3.02 (dd, J = 15.6, 7.0 Hz, 1 H), 2.95-2.82 (m, 2H), 2.77 - 2.69 (m, 1 H), 2.42-2.30 (m, 1 H), 2.03-190 (m, 1H), 1.76-1.63 (m, 1H), 1.41-1.30 (m, 4H), 1.19 (s, 3H), 1.13 (s, 3H) and 0.76 (s, 3H).

This cocrystal Form B may also be further characterized by a DSC curve which shows a sharp endothermic peak with onset at approximately 156.5 °C, caused by the melting of the Form B. The DSC analysis indicates that Form B is an anhydrous form.

This cocrystal of (R)-Lorcaserin hydrochloride with (+)-camphoric acid can be prepared by a process which comprises wet grinding (R)-Lorcaserin hydrochloride hemihydrate Form III, (1 R,3S)-(+) camphoric acid, and a solvent selected from the group consisting of methanol, acetonitril, and ethyl acetate; and isolating the solid thus obtained.

It can also be prepared by a process which comprises crystallizing the cocrystal from a solution of (R)-Lorcaserin hydrochloride, (1 R, 3S)-(+)-camphoric acid, and isobutyl acetate; and isolating the solid thus obtained.

Finally, it can also be prepared by a process which comprises slurrying a mixture of (R)-Lorcaserin hydrochloride hemihydrate Form III, (1 R,3S)-(+) camphoric acid, and a solvent selected from ethyl acetate and methylisobutylketone; and isolating the solid thus obtained.

These processes afford the cocrystal of (R)-Lorcaserin hydrochloride with (1 R, 3S)-(+)-camphoric acid Form B.

The cocrystal of (R)-Lorcaserin hydrochloride with (+)-camphoric acid Form B of the invention may also be defined by its preparation process. Accordingly, this aspect of the invention can be formulated as cocrystal of (R)-Lorcaserin hydrochloride with (+)-camphoric acid Form B as defined above, obtainable by any of the previous processes, optionally including any preferred or particular embodiment of the process and possible combinations of some of the process features disclosed above.

It is well known that the formation of pharmaceutical cocrystal allows tayloring physical-chemical properties of an API such as solubility in water or organic solvents, the amount of residual solvent, purification, crystal morphology (filtration, flowability and density), compressibility or hygroscopicity. Advantageously, the cocrystal of (R)-Lorcaserin hydrochloride with (+)-camphoric acid Form B is an anhydrous non-hygroscopic form.

In another particular embodiment the cocrystal of Lorcaserin hydrochloride and a organic diacid is a cocrystal of (R)-Lorcaserin hydrochloride with oxalic acid.

In a preferred embodiment of the invention, the cocrystal of (R)-Lorcaserin hydrochloride with oxalic acid has a molar ratio of (R)-Lorcaserin hydrochloride/oxalic acid of 2:1 (calculated by elemental analysis of a sample).

| | (R)-LorcaserineHCl·oxalic acid (2:1) | | | |
|---|---|---|---|---|
| Element | Cl (%) | N (%) | C (%) | H (%) |
| Calculated | 25.63 | 5.05 | 51.98 | 5.77 |
| Experimental | 25.83 | 5.08 | 51.64 | 5.73 |

In a more preferred embodiment, the cocrystal of (R)-Lorcaserin hydrochloride with oxalic acid is characterized by having an X-ray diffractogram that comprises characteristic peaks at approximately 13.9 and 17.1 degrees 2 theta (Cu-K_{α} radiation, λ = 1.5406 Å). This specific cocrystal form is named herein cocrystal Form C. In a still more preferred embodiment, the cocrystal Form C is characterized by having an X-ray diffractogram that further comprises characteristic peaks at approximately 14.6 and 21.6 degrees 2 theta (Cu-K_{α} radiation, λ = 1.5406 Å). More specifically, this new cocrystal Form C is characterized by exhibiting in the powder X-ray diffractogram a pattern of peaks, expressed in 2 theta units in degrees, 2θ (°), which is shown in Table 3.

**Table 3: List of selected peaks of XRPD (only peaks with relative intensity greater than or equal to 1% are indicated):**

| Pos. | Rel. |
|---|---|
| [°2Th.] | Int. [%] |
| 6,1 | 22 |
| 12,3 | 45 |
| 12,6 | 9 |
| 13,2 | 53 |
| 13,9 | 55 |
| 14,6 | 73 |
| 15,2 | 6 |
| 16,5 | 10 |
| 17,1 | 100 |
| 18,4 | 20 |
| 19,1 | 12 |
| 20,2 | 20 |
| 20,4 | 13 |
| 20,9 | 4 |
| 21,6 | 20 |
| 23,0 | 3 |
| 23,3 | 25 |
| 23,7 | 37 |
| 24,0 | 28 |
| 24,8 | 6 |
| 25,5 | 26 |
| 25,8 | 8 |
| 26,1 | 7 |
| 26,6 | 27 |
| 26,8 | 18 |
| 27,2 | 4 |
| 28,1 | 36 |
| 29,0 | 12 |
| 29,5 | 3 |
| 30,7 | 13 |
| 30,9 | 12 |
| 31,1 | 11 |
| 31,5 | 34 |
| 31,6 | 34 |
| 33,3 | 9 |
| 34,3 | 9 |
| 34,5 | 15 |
| 35,4 | 2 |
| 36,7 | 8 |
| 37,5 | 5 |
| 38,1 | 8 |
| 38,7 | 8 |
| 38,9 | 3 |

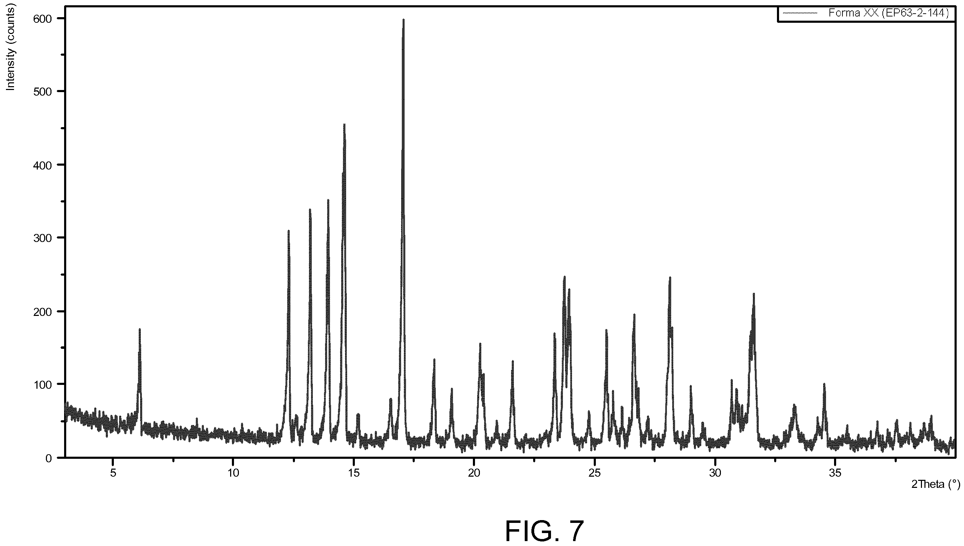

The cocrystal Form C may be further characterized by an X-ray diffractogram as in FIG. 7.

The cocrystal Form C may also be further characterized by the following ¹H NMR (DMSO-d6, 400 MHz): δ = 9.57 (s broad, 1H), 9.15 (s broad, 1H), 7.29-7.20 (m, 3H), 3.52-3.39 (m, 1H), 3.37-3.15 (m, 3H), 3.02 (dd, J = 15.6, 7.0 Hz, 1H), 2.96-2082 (m, 2H) and 1.35 (d, J= 7.0 Hz, 3H).

The cocrystal Form C may also be further characterized by the following ¹³C NMR (DMSO-d6, 400 MHz):δ = 161.1, 145.4, 138.1, 131.6, 126.5, 126.1, 50.2, 44.7, 34.3, 30.9 and 17.6.

The cocrystal Form C may be further characterized by a DSC curve which shows three endothermic events. The last one with an onset at 115.20°C is caused by the melting of the Form C.

This cocrystal of R)-Lorcaserin hydrochloride with oxalic acid can be prepared by a process which comprises: a1) mixing (R)-Lorcaserin free base and methylisobutylketone; b1) adding oxalic acid followed by adding hydrochloric acid in ethyl acetate; and c1) isolating the solid thus obtained.

This process afford the cocrystal of (R)-Lorcaserin hydrochloride with oxalic acid Form C.

The cocrystal of (R)-Lorcaserin hydrochloride with oxalic acid Form C of the invention may also be defined by its preparation process. Accordingly, this aspect of the invention can be formulated as cocrystal of (R)-Lorcaserin hydrochloride oxalic acid Form C as defined above, obtainable by any of the previous processes, optionally including any preferred or particular embodiment of the process and possible combinations of some of the process features disclosed above.

It is part of the invention, the cocrystals of Lorcaserin hydrochloride and an organic diacid of the present invention for use as a medicament.

It is also part of the invention the cocrystals of Lorcaserin hydrochloride and an organic diacid of the present invention for use in the prevention and/or treatment of obesity.This aspect can also be formulated as the use of cocrystals of (R)-Lorcaserin hydrochloride and an organic diacid of the present invention for the preparation of a medicament for the prophylactic and/or therapeutic treatment of obesity in a mammal, including a human. The invention also relates to a method of treatment and/or prophylaxis of a mammal, including a human, suffering from or being susceptible to suffer obesity, said method comprises the administration to said patient of a therapeutically effective amount of any of the cocrystals of (R)-Lorcaserin hydrochloride and an organic diacid of the present invention, together with pharmaceutically acceptable excipients or carriers.

Any of the cocrystals of (R)-Lorcaserin hydrochloride and a pharmaceutically acceptable organic diacid of the invention disclosed above may be employed in various pharmaceutical formulations. Accordingly, a pharmaceutical composition comprising a cocrystal of cocrystals of (R)-Lorcaserin hydrochloride and an organic diacid, together with appropriate amounts of pharmaceutical excipients or carriers is also part of the invention. The pharmaceutical composition may be used for oral administration. Examples of the pharmaceutical composition for oral administration include tablets, pills, capsules, granules or powders. Appropriate excipients for the pharmaceutical composition include diluents, disintegrants, lubricants or and/or binders. If desired, the tablets may be coated with sugar coat or with gastric or enteric coating.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### Example 1: Preparation process of a cocrystal of (R)-Lorcaserin hydrochloride with (+)-di-p-toluoyl-D-tartaric acid Form A by crystallization in water saturated MIBK

In a glass tube of 25 mL, racemic Lorcaserin hydrochloride (1.00 g, 4.31 mmol) and (+)-dip-toluoyl-D-tartaric acid (1.67 g, 4.31 mmol, 1.0 equiv.) were suspended methylisobutyl ketone (MIBK) saturated of water (5 mL, 5v). Then, the suspension was heated to reflux. The resulting opalescent solution was slowly cooled to room temperature. The suspension was stirred 30 minutes at room temperature and 30 minutes at 0-5°C in an ice bath. The white solid was filtered in a sintered funnel (no.3), washed twice cold MIBK saturated of water (2 x 1 mL, 2 x 1 v) and dried at RT under high vacuum. 831 mg of Form A (46 % yield) were obtained with 72% ee.

### Example 2: Preparation of the cocrystal of (R)-Lorcaserin hydrochloride with (+)-di-p-toluoyl-D-tartaric acid Form A

In a round bottom 100 mL flask, racemic Lorcaserin hydrochloride (5.00 g, 21.5 mmol) and (+)-di-p-toluoyl-D-tartaric acid (16.64 g, 43.1 mmol, 2.0 equiv.) were suspended in methyl ethyl ketone (25 mL, 5 v) before adding water (0.15 mL, 0.03 v) and heating to reflux. The resulting clear solution was stirred for 15 minutes at reflux before cooling slowly to room temperature. During the cooling the solution was seeded with Form A and a white solid progressively crystallized. Then, the resulting suspension was stirred 30 minutes at room temperature and 30 minutes at 0-5°C with an ice bath before isolating the solid. The white solid was filtered in a sintered funnel (no.3), washed twice with cold MEK/water mixture (2 x 5 mL, 2 x 1 v, same ratio as reaction solvent) and dried at high vacuum. 3.68 g of Form A (40% yield) were obtained with 80% ee.

### Example 3: Recrystallization of (R)-Lorcaserin hydrochloride with (+)-di-p-toluoyl-D-tartaric acid Form A

In a round bottom 25 mL flask, Form A (1.22 g, 1.43 mmol), previously obtained, was dissolved in isobutyl alcohol (4.9 mL, 4 v) at reflux before adding dropwise heptane (7.3 mL, 6 v). The resulting suspension was stirred for 15 minutes at reflux before cooling slowly to room temperature. Then, the suspension was stirred 30 minutes at room temperature and 30 minutes at 0-5°C with an ice bath. The white solid was filtered with a sintered funnel (no.3), washed twice with cold isobutanol/heptane 4:6 mixture (1.2 mL, 1 v) and dried at RT under high vacuum. 1.02 g of Form A (84% yield) were obtained with 95.4% ee.

### Example 4: Second recrystallization of the cocrystal of (R)-Lorcaserin hydrochloride with (+)-di-p-toluoyl-D-tartaric acid Form A

In a round bottom 25 mL flask, Form A (1.01 g, 1.18 mmol) previously obtained in the experiment EP63-2-108A was dissolved in isobutyl alcohol (4.0 mL, 4 v) before adding dropwise heptane (6.1 mL, 6v) the resulting suspension was stirred for 15 minutes under reflux before cooling slowly to room temperature. Then, the suspension was stirred 30 minutes at room temperature and 30 minutes at 0-5°C with an ice bath. The white solid was filtered in a sintered funnel (no.3), washed twice with cold isobutanol/heptane 4:6 mixture (1 mL, 1 v) and dried at RT under high vacuum. 0.89 g of Form A (88% yield) were obtained with 99.0% ee.

### Example 5: Preparation of the cocrystal of (R)-Lorcaserin hydrochloride and camphoric acid Form B by wet grinding

In an Eppendorfwere added (R)-Lorcaserin hydrochloride hemihydrate Form III (30.0 mg, 0.129 mmols), (1R, 3S)-(+) camphoric acid (25.9 mg, 0.129 mmols, 1 eq.), two drops of MeOH and three steel balls. The Eppendorf was stirred at 30 Hz for 15 minutes (3 cycles). The resulting mixture was dried under high vacuum and analyzed by XRPD. Pure Form B was obtained.

The same experiment using two drops of ACN or EtOAc afforded the same results.

### Example 6: Preparation of the cocrystal of (R)-Lorcaserin hydrochloride and camphoric acid by crystallization in isobutyl acetate

In a test tube were added (R)-Lorcaserin hydrochloride (86.6 mg, 0.373 mmols), (1 R, 3S)-(+)-camphoric acid (148.9 mg, 0.744 mmols, 2 eq.) and isobutyl acetate (0.86 mL, 10v). Then, the resulting mixture was stirred for 15 minutes under reflux before cooling slowly to room temperature. Then, the suspension was stirred at room temperature overnight. The resulting solid was filtered in a sintered funnel (no.4), washed with cold isobutanol (0.5 mL) and dried at RT under high vacuum. 130.9 mg of Form B (81% yield) were obtained.

### Example 7: Preparation of cocrystal of (R)-Lorcaserin hydrochloride and camphoric acid by slurring in ethyl acetate

In a test tube were added (R)-Lorcaserin hydrochloride hemihydrate Form III (49.4 mg, 0.205 mmols), (1R, 3S)-(+)-camphoric acid (124.6 mg, 0.622 mmols, 3 eq.) and ethyl acetate (0.5 mL, 10v). Then, the mixture was stirred at room temperature overnight. The resulting solid was recovered by filtration and dried at RT under high vacuum. The solid was confirmed to be Form B by XRPD.

### Example 8: Preparation of the cocrystal of (R)-Lorcaserin hydrochloride and camphoric acid Form B from the cocrystal of (R)-Lorcaserin hydrochloride with (+)-di-p-toluoyl-D-tartaric acid Form A

In a round botton 10 mL flask, cocrystal of (R)-Lorcaserin hydrochloride with (+)-di-p-toluoyl-D-tartaric acid Form A (300.2 mg, 0.353 mmol) was dissolved in a biphasic mixture of ethyl acetate / water (1:1, 20 vol.). Then the aqueous phase was recovered and basified with sodium hydroxide 1 M to reach pH 10 and the resulting mixture was extracted with methyl isobutyl ketone (3 x 3 mL, 3 x 10 v). The organic phase was evaporated under vacuum to dryness. The resulting oil was dissolved in methyl isobutyl ketone (0.5 mL, 1.67 v) before adding successively (1 R, 3S)-(+)-camphoric acid (424.6 mg, 2.118 mmols) and 704 µL (0.704 mmol) of hydrochloric acid 1 M dissolved in ethyl acetate. The reaction mixture was seeded with the Form B and was left stirring at room temperature until a solid precipitated. Then, the reaction mixture was cooled in an ice bath for 1 hour. The solid was recovered by filtration, washed twice with cold methyl isobutyl ketone (0.1 mL, 0.33 v) and dried at RT under high vacuum. 224.8 mg of cocrystal of (R)-Lorcaserin hydrochloride and camphoric acid Form B (74% yield) were obtained as confirmed by XRPD. HPLC revealed an ee of 99.0%.

### Example 9: Preparation of the cocrystal of (R)-Lorcaserin hydrochloride with oxalic acid Form C by crystallization in MIBK saturated of water

In a test tube, (R)-Lorcaserin hydrochloride hemihydrate (49.7 mg, 0.206 mmol) and oxalic acid (37.7 mg, 0.419 mmol, 2 equiv.) were suspended in MIBK saturated of water (0.25 mL, 5 v). Then, the resulting mixture was heated to reflux before seeding with form XIX (salt of (R)-Lorcaserin base and oxalic acid which can be obtained by slurrying in ethyl acetate, MIBK, ACN or ethyl methyl ketone of a 1:2 mixture; having an X-ray diffractogram that comprises characteristic peaks at approximately 6.2, 14.1, 14.4 and 21.5 degrees 2 theta (Cu-K_{α} radiation, λ = 1.5406 Å)). Then, the solution was slowly cooled to room temperature and a solid crystallized. It was recovered by centrifugation and was dried at RT under high vacuum. The formation of cocrystal Form C, with an excess of oxalic acid, was confirmed by XRPD.

### Example 10: Preparation of (R)-Lorcaserin hydrochloride with oxalic acid Form C by crystallization without seeding

In a test tube were added (R)-Lorcaserin hydrochloride (49.7 mg, 0.206 mmols), oxalic acid (38.8 mg, 0.431 mmols) and methyl isobutyl ketone (0.1 mL, 2 v). Then, the resulting mixture was heated to reflux until obtained complete dissolution. The clear solution was cooled slowly to room temperature before adding heptane (0.25 mL, 0.5 v). The precipitated solid was recovered by filtration and dried at RT under high vacuum. The formation of cocrystal Form C, with an excess of oxalic acid, was confirmed by XRPD.

### Example 11: Preparation of the cocrystal Form C from (R)-Lorcaserin base

In a round bottom 10 mL flask, (R)-Lorcaserin hydrochloride (300 mg, 1.244 mmol) was dissolved in water (2.0 mL, 6.67 v.). Then, sodium hydroxide 1 M was added to the mixture to reach pH 10. The aqueous phase was extracted with methyl isobutyl ketone (3 x 2.0 mL, 3 x 6.67 v). The organic phase was evaporated under vacuum to dryness. The resulting oil was dissolved in methyl isobutyl ketone (2 mL, 6.67 v.) before adding successively oxalic acid (224.5 mg, 2.494 mmols) and 1.25 mL (1.25 mmol) of hydrochloric acid 1 M dissolved in ethyl acetate. Then, the reaction mixture was seeded with the Form C and was left stirring at room temperature overnight. The solid was recovered by filtration, washed twice with methyl isobutyl ketone (2 x 0.25 mL, 2 x 0.83v) and dried at RT under high vacuum. 271.2 mg of Form C (68% yield) were obtained as confirmed by XRPD.

Same procedure has been carried out without seeding affording Form C with similar yield.

### REFERENCES CITED IN THE APPLICATION

- EP1411881B1
- EP2189448B1
- WO2015007897A1
- US8168782B2
- CN103755635
- EP2327698B1
- WO2014135545A1
- WO2011153206A1

## Claims

1. A cocrystal of (R)-Lorcaserin hydrochloride and an organic diacid.

2. The cocrystal according to claim 1, wherein the organic diacid is selected from the group consisting of: (+)-di-p-toluoyl-D-tartaric acid; (+)-camphoric acid; and oxalic acid.

3. The cocrystal according to any of the claims 1-2, which has a molar ratio of (R)-Lorcaserin hydrochloride / organic diacid of 1:1 or 2:1.

4. The cocrystal according to any of the claims 1-3, which is a crystalline form of the cocrystal of Lorcaserin hydrochloride with (+)-di-p-toluoyl-D-tartaric acid named Form A and is **characterized by** having an X-ray diffractogram that comprises characteristic peaks at approximately 6.8 and 13.5 degrees 2 theta (Cu-K_{α} radiation, λ = 1.5406 Å).

5. The cocrystal according to any of the claims 1-3, which is a crystalline form of the cocrystal of (R)-Lorcaserin hydrochloride with (+)-camphoric acid named Form B and is **characterized by** having an X-ray diffractogram that comprises characteristic peaks at approximately 14.2 and 18.7 degrees 2 theta (Cu-K_{α} radiation, λ = 1.5406 Å).

6. The cocrystal according to any of the claims 1-3, which is a crystalline form of the cocrystal of (R)-Lorcaserin hydrochloride with oxalic acid named Form C and is **characterized by** having an X-ray diffractogram that comprises characteristic peaks at approximately 13.9 and 17.1 degrees 2 theta (Cu-K_{α} radiation, λ = 1.5406 Å).

7. A cocrystal of Lorcaserin hydrochloride and an organic diacid as defined in any of the claims 1-6, for use as a medicament.

8. A cocrystal of Lorcaserin hydrochloride and an organic diacid as defined in any of the claims 1-6, for use in the prevention and/or treatment of obesity.

9. A pharmaceutical composition comprising a cocrystal of Lorcaserin hydrochloride and an organic diacid as defined in any of the claims 1-6, together with appropriate amounts of pharmaceutical excipients or carriers.

10. A preparation process of a cocrystal of Lorcaserin hydrochloride with (+)-di-p-toluoyl-D-tartaric acid, which comprises:
a1) either crystallizing/precipitating the cocrystal from a mixture of racemic Lorcaserin hydrochloride or (R)-Lorcaserin hydrochloride, (+)-di-p-toluoyl-D-tartaric acid, and a solvent system selected from the group consisting of: methylethylketone/water, acetone/water, isobutanol, n-butanol, ethyl acetate, and methylisobutylketone saturated of water, or
a2) slurrying racemic Lorcaserin hydrochloride or (R)-Lorcaserin hydrochloride with (+)-dip-toluoyl-D-tartaric acid in ethyl acetate at room temperature; and
b) isolating the solid thus obtained in steps a1) or a2).

11. The process according to claim 10, wherein the cocrystal of Lorcaserin hydrochloride with (+)-di-p-toluoyl-D-tartaric acid is cocrystal Form A as defined in claim 4, and wherein the process further comprises carrying out one or two recrystallizations of the cocrystal of Lorcaserin hydrochloride with (+)-di-p-toluoyl-D-tartaric acid in a solvent system selected from the group consisting of isobutanol, isobutanol/antisolvent, n-butanol, and n-butanol/antisolvent, wherein the antisolvent is selected from the group consisting of isobutyl acetate, heptane, methylisobutylketone, tert-butyl methyl ether, and mixtures thereof.

12. A resolution process of Lorcaserin hydrochloride comprising:
a) preparing a cocrystal of Lorcaserin hydrochloride with (+)-di-p-toluoyl-D-tartaric acid Form A, by a procees which comprises either crystallizing the cocrystal from a mixture of racemic Lorcaserin hydrochloride and (+)-di-p-toluoyl-D-tartaric acid from a solvent system selected from the group consisting of: methylethylacetone/water, acetone/water, isobutanol, n-butanol, ethyl acetate, and methylisobutylketone saturated of water;
or alternatively, precipitating the cocrystal from a mixture of racemic Lorcaserin hydrochloride and (+)-di-p-toluoyl-D-tartaric acid from methylisobutylketone saturated of water;, followed by isolating the solid thus obtained; and carrying out one or two recrystallizations in a solvent system selected from the group consisting of n-Butanol, isobutanol, n-Butanol/antisolvent and isobutanol/antisolvent, wherein the antisolvent is selected from the group consisting of isobutyl acetate, heptane, methylisobutylketone, tert-butyl methyl ether, and mixtures thereof;
b) slurrying the cocrystal in a biphasic solvent system selected from the group consisting of ethyl acetate/water, and methylisobutylketone/water to dissociate the cocrystal Form A; and
c) isolating (R)-Lorcaserin hydrochloride from the aqueous phase.

13. Use of the cocrystal of (R)-Lorcaserin hydrochloride with (+)-di-p-toluoyl-D-tartaric acid Form A as an intermediate for the preparation of (R)-Lorcaserin or a salt thereof with ee ≥ 99%, or of cocrystals of (R)-Lorcaserin hydrochloride and a organic diacid with ee ≥ 99%.

14. A process for preparing a cocrystal of (R)-Lorcaserin hydrochloride with (+)-camphoric acid which comprises:
a1) either wet grinding (R)-Lorcaserin hydrochloride hemihydrate Form III, (1 R,3S)-(+) camphoric acid, and a solvent selected from the group consisting of methanol, acetonitril, and ethyl acetate; or
a2) crystallizing the cocrystal from a solution of (R)-Lorcaserin hydrochloride, (1 R, 3S)-(+)-camphoric acid, and isobutyl acetate; or
a3) slurrying a mixture of (R)-Lorcaserin hydrochloride hemihydrate Form III, (1 R,3S)-(+) camphoric acid, and a solvent selected from ethyl acetate and methylisobutylketone; and
b) isolating any of the solids obtained in steps a1), a2) or a3).

15. A process for preparing a cocrystal of (R)-Lorcaserin hydrochloride with oxalic acid which comprises:
a1) mixing (R)-Lorcaserin free base and methylisobutylketone;
b1) adding oxalic acid, followed by adding hydrochloric acid in ethyl acetate; and
c1) isolating the solid thus obtained.
